(19) Europäisches Patentamt
European Patent Office
Office européen des brevets

(11) Veröffentlichungsnummer: **0 257 344**
**B1**

# (12) EUROPÄISCHE PATENTSCHRIFT

(45) Veröffentlichungstag der Patentschrift: **16.01.91**

(51) Int. Cl.$^5$: **A 61 K 31/62**

(21) Anmeldenummer: **87111104.3**

(22) Anmeldetag: **31.07.87**

(54) **Arzneimittel enthaltend Dipyridamol oder Mopidamol und O-Acetylsalicylsäure bzw. deren physiologisch verträgliche Salze, Verfahren zu ihrer Herstellung und ihre Verwendung zur Bekämpfung der Thrombusbildung.**

(30) Priorität: **13.08.86 DE 3627423**

(43) Veröffentlichungstag der Anmeldung:
**02.03.88 Patentblatt 88/09**

(45) Bekanntmachung des Hinweises auf die Patenterteilung:
**16.01.91 Patentblatt 91/03**

(84) Benannte Vertragsstaaten:
**AT BE CH DE ES FR GB GR IT LI LU NL SE**

(56) Entgegenhaltungen:
**EP-A-0 169 465**
**FR-A-2 368 280**
**FR-A-2 368 272**
**FR-A-2 390 959**

**UNLISTED DRUGS, Band 29, Nr. 11, November 1977, Seite 177q, Chatham, New Jersey, US; "Asasantin"**

**Die Akte enthält technische Angaben, die nach dem Eingang der Anmeldung eingereicht wurden und die nicht in dieser Patentschrift enthalten sind.**

(73) Patentinhaber: **Dr. Karl Thomae GmbH**
**Postfach 1755**
**D-7950 Biberach (Riss) (DE)**

(72) Erfinder: **Eisert, Wolfgang, Prof. Dr. Dr. Dr.**
**Friedrich-Goll-Weg 5**
**D-7950 Biberach 1 (DE)**
Erfinder: **Gruber, Peter, Dr.**
**Friedrich-Ebert-Strasse 70**
**D-7950 Biberach 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 257 344 B1

Courier Press, Leamington Spa, England.

**Beschreibung**

Die Erfindung betrifft Arzneimittelkombinationen, bestehend aus Dipyridamol oder Mopidamol und O-Acetylsalicylsäure bzw. deren physiologisch verträglichen Salzen, Verfahren zur Herstellung dieser Arzneimittelkombinationen und deren Verwendung der gezielten Verhütung von Thrombusbildungen.

Von O-Acetylsalicylsäure ist bekannt, daß es als Hemmstoff der Aggregation menschlicher Blutplättchen entgegenwirkt (vgl. Br. J. Clin. Pharmac. *7* (1979) 283). Es wurde berichtet, daß O-Acetylsalicylsäure das Enzym Cyclooxygenase in den Blutplättchen und damit die Biosynthese des die Aggregation fördernden Thromboxan $A_2$ hemmt. Mit steigender Dosierung nimmt zwar die antithrombotische Wirkung der O-Acetylsalicylsäure zu, gleichzeitig aber auch die hemmende Wirkung auf die Cyclooxygenase der Gefäßwände, wodurch mittelbar auch die Synthese des die Aggregation hemmenden Prostazyklins negativ beeinflußt wird. Es wird nahegelegt (vgl. Lancet, III (1979) 1213, Prostaglandins and Medicine *4* (1980) 439) mit möglichst niedrigen Dosierungen an Acetylsalicylsäure auszukommen. Andererseits wird aber empfohlen (Prostaglandins, Leukotriens und Medicin *12* (1983) 235), höhere Dosierungen zu verwenden, da mit steigender Dosierung die antithrombotische Wirkung der Acetylsalicyclsäure auch dann noch erhöht wird, wenn die Prostazyklin- und Thromboxan-Biosynthese bereits gehemmt wird.

Dipyridamol (2,6-Bis-(diäthanolamino)-4,8-dipiperidino-(5,4-d)-pyrimidin) und Mopidamol (2,6-Bis-(diäthanolamino)-8-piperidino-(5,4-d)-pyrimidin) werden klinisch unter anderem als antithrombotisch und antiaggregatorisch wirkende und Mopidamol auch als Metastasen-inhibierende Wirkstoffe eingesetzt.

Durch die FR—B—2 368 272 wird eine Kombination aus Dipyridamol und O-Acetylsalicylsäure, vorzugsweise in einem Gewichtsverhältnis von 4:1 bis 1:4, beschrieben, welche eine synergistische Wirkung auf die induzierte Thrombocytenaggregation ausübt. Da die beiden Komponenten miteinander chermisch unverträglich sind, wurde vorgeschlagen, diese räumlich voneinander zu trennen, z.B. durch Schaffung sogenannter Schichttabletten oder Mantel-Kern-Tabletten.

Bekannt ist das Präparat Asasantin® der Firma Dr. Karl Thomae GmbH, Biberach/Riss, enthaltend 330 mg O-Acetylsalicylsäure neben 75 mg Dipyridamol, bekannt wurden auch Kombinationsmöglichkeiten anderer Pyrimidopyrimidine mit der Acetylsalicylsäure in Verhältnissen von 0,5 und darunter (vgl. FR—B—2 390 959).

Die DE—A1—3 515 874 beschreibt Kombinationspräparate, enthaltend Pyrimido-pyrimidine, insbesondere Dipyridamol und/oder Mopidamol und O-Acetylsalicylsäure oder Salze dieser Stoffe, wobei das Gewichtsverhältnis der Pyrimido-pyrimidin-Komponente zu der O-Acetylsalicylsäure-Komponente größer als 0,5 ist, und die Pyrimido-pyrimidin-Komponente zuerst freigesetzt (bioverfügbar) wird. Dies kann, z.B. dadurch erreicht werden, daß pharmazeutische Träger und Hilfsstoffe verwendet werden, die für eine zeitlich abgestufte Freisetzung der beiden Komponenten sorgen. Nach den dort gemachten Angaben läßt sich der medizinische Effekt nur dann erzielen, wenn der relative Acetylsalicylsäure-Anteil bei der zeitlich aufeinanderfolgenden (sequentiellen bzw. konsekutiven) Gabe so gewählt ist, daß ein Gewichtsverhältnis von Pyrimido-pyrimidin zu O-Acetylsalicylsäure von 0,5 nicht unterschritten wird. Das Gewichtsverhältnis von Pyrimido-pyrimidin zu O-Acetylsalicylsäure soll mehr als 0,5 und bis zu 30 betragen. Ganz bevorzugt soll es zwischen 0,6 und 3 liegen. Zwischen der Freisetzung der Pyrimido-pyrimidin-Komponente und der O-Acetylsalicylsäure-Komponente muß ein Zeitabstand von 15 Minuten bis zu 2 Stunden, vorzugsweise von 30 Minuten bis zu 90 Minuten liegen, ganz bevorzugt zwischen 40 und 70 Minuten. Nach den dortigen Angaben wird es, wegen eines überadditiven Effektes, möglich, die Dosierung der Einzelkomponenten entschiedend zu erniedrigen, und zwar weit unter die Dosierungen, die man zur Erzielung des gleichen Effektes mit Einzelgaben Acetylsalicylsäure oder Pyrimido-pyrimidin oder mit der kombinierten gleichzeitigen Verabreichung von Acetylsalicylsäure und Pyrimido-pyrimidin benötigen würde.

Es wurde nun gefunden, daß eine Kombination bestehend aus Dipyridamol und/oder Mopidamol bzw. deren physiologisch verträglichen Salzen und O-Acetylsalicylsäure bzw. ihrer physiologisch verträglichen Salzen, welche die beiden Komponenten in einem Gewichtsverhältnis von 8:1 und größer enthält und die beiden Komponenten gleichzeitig im Magen-Darm-Trakt freisetzt, die Thrombusbildung signifikant reduziert bzw. verhindert und gleichzeitig einen einmal gebildeten Thrombus schneller auflöst, als dieses durch die natürliche Thrombolyse der Fall wäre. In manchen Fällen, die von der Ursache der Thrombusbildung abhängen, kann es sogar von Vorteil sein, wenn einer solchen Kombination zuerst die O-Acetylsalicylsäure und, zeitlich versetzt, das Pyrimido-pyrimidin im Magen-Darm-Trakt freigesetzt werden. Als Zeitabstand kommen hierfür 15 bis 90 Minuten in Frage. Als oberer Grenzwert für das Verhältnis von Pyrimido-pyrimidin zu O-Acetylsalicylsäure kann, in erster Linie aus praktischen Gründen, die Zahl 100 angesehen werden, diese Obergrenze ist aber für das Zusammenwirken der beiden Komponenten als nicht kritsch zu betrachten. Eine Begrenzung des Pyrimido-pyrimidin-Gehalts ist z.B. für schluckbare Arzneiformen, wie Tabletten oder Dragées, dadurch gegeben, daß bei einer weiteren Erhöhung des Pyrimido-pyrimidin-Gehalts diese Formen zu voluminös werden.

Die beiden Komponenten Pyrimido-pyrimidin und O-Acetylsalicylsäure können als ein Gemisch vorliegen, welches sich für die Herstellung von Instant-Formen besonders eignet, wobei die beiden Komponenten durch Anbringung einer geeigneten Schutzschicht voneinander getrennt und damit lagerstabil sind. Bekanntlich ist die Komponente O-Acetylsalicylsäure nicht frei von Spuren an Essigsäure, die aus der Spaltung der Acetylsalicylsäure während der Lagerung entstehen. Die freie Essigsäure reagiert mit dem Dipyridamol unter Bildung hygroskopischer Salze und Dipyridamol-Essigsäure-Ester, die zum Verderb des Dipyridamols führen. Man kann diese Vorgänge am Dipyridamol dadurch unterbinden, daß man die eine oder die andere oder beide Komponenten mit einer Trennschicht versieht. So versieht man z.B. Dipyrimadol in Form von Pellets oder Granulaten mit einem magensaftunlöslichen, aber darmsaftlöslichen Lack und/oder die Acetylsalicylsäure-Kerne oder -Tabletten mit einem essigsäuredichten Überzug, der im Magensaft sehr schnell aufgelöst wird. Gleiches gilt auch für das Mopidamol.

Man kann aber auch beispeilsweise ein Dipyridamolgranulat, ein Trenngranulat und ein Acetylsalicylsäuregranulat getrennt herstellen und anschließend zu Dreischicht-Tabletten verpressen; ein Pyrimido-pyrimidin-Granulat läßt sich aber auch, zusammen mit einem eine Schutzschicht tragenden Dragée bzw. Filmtablette, enthaltend die O-Acetylsalicylsäure, in eine Kapsel einfüllen. Legt man dagegen einen besonderen Wert auf einen gleichmäßig hohen Pyrimido-pyrimidinblutspiegel, so geht man vorteilhafterweise von Pyrimido-pyrimidin-Pellets aus, welche eine zeitlich und pH-gesteuerte Abgabe dieses Wirkstoffes ermöglichen, und verarbeitet diese zusammen mit der O-Acetylsalicylsäure zu entsprechenden Arzneiformen. Hierbei kann es such vorteilhaft sein, Preßlinge der O-Acetylsalicylsäure mit einer entsprechenden Abdeckung durch eine Filmschicht herzustellen und diese mit den Pyrimido-pyrimidin-Pellets zu kombinieren. Will man eine zeitlich vorversetzte Abgabe der O-Acetylsalicylsäure erreichen, so kann man die Pyrimido-pyrimidin-Pellets mit einer, die Abgabe dieses Wirkstoffes retardierenden Schicht und die O-Acetylsalicylsäure enthaltende Kerne mit einer magensaftlöslichen Schicht überziehen. Im Falle von Dipyridamol-Pellets mit einer kontrollierten Freigabe des Wirkstoffes ist es besonders vorteilhaft, solche zu verwenden, welche gemäß den in der DE—A—3 000 979.1 beschriebenen Rezepturen hergestellt wurden. Diese Pellets besitzen einen Überzug, der als Dialysemembran wirkt und den Wirkstoff Dipyridamol in Verbindung mit Säuren im Magen-Darm-Trakt in geregelter Weise retardiert abgibt. Der Aufbau und die Zusammensetzung der Pellets führt zur Freigabe gelösten Dipyridamols in Form seiner Salze. So hergestellte Retard-Pellets führen zu einer vollständigen Resorption des Dipyridamols aus dem Magen-Darm-Trakt. Man erreicht damit gleichmäßige Blutspiegel über 8 bis 10 Stunden unter Vermeidung von Blutspiegelspitzen, wie sie bei den sich im Handel befindlichen Dipyridamol-Acetylsalicylsäure-Formen (z.B. gemäß FR—B—2 368 272) häufig auftreten. Die bei den Formen gemäß dieser französischen Patentschrift beobachteten Blutspiegelspitzen rühren davon her, daß Dipyridamol nur teilweise und individuell in verschieden hohem Maße resorbiert wird. Die Blutspiegelwerte fallen bei manchen Patienten nach kurzer Zeit unter den wirksamen Bereich ab bzw. werden zum Teil nicht erreicht (bei einem sogenannten "non-absorber"); die O-Acetylsalicylsäure erreicht dagegen bei jedem Patienten ihre pharmakologische Wirksamkeit.

Ein bevorzugter Gegenstand der vorliegenden Erfindung betrifft also Arzneimittelformen, bestehend aus Dipyridamol und/oder Mopidamol in Verbindung mit einer physiologisch verträglichen Säure, wobei mindestens 1 Val Säure auf 1 Mol Dipyridamol oder Mopidamol kommen, umgeben von einer Umhüllung, die aus 50 bis 100% säureunlöslichen, darmsaftlöslichen Lacken und aus 50 bis 0% magen- und darmsaftunlöslichen Lacken besteht, wobei diese Komponente in Form von Granulaten oder Pellets vorliegt, und der Komponente O-Acetylsalicylsäure, die vorzugsweise mit einem magensaftlöslichen Überzug versehen ist und vorzugsweise als Dragée oder Filmtablette vorliegt, wobei die beiden Komponenten in einem Gewichtsverhältnis von mindestens 8 vorliegen müssen.

Während bevorzugte Formen gemäß der DE—A1—3 515 874 ein Gewichtsverhältnis von Pyrimido-pyrimidin zu O-Acetylsalicylsäure zwischen 0,6 und 1.5 besitzen (darüber hinausgehende Verhältnisse sind nicht durch Beispiele beschrieben), betragen diese Gewichtsverhältnisse im Falle der vorliegenden Erfindung 8 und größer vorzugsweise 8 bis 100.

Hierbei enthalten die Arzneimittelformen zwischen 10 und 675 mg Dipyridamol und/oder Mopidamol und zwischen 1 und 150 mg O-Acetylsalicylsäure. Für Dipyridamol und Mopidamol bevorzugte Formen enthalten zwischen 75 und 400 mg dieses Wirkstoffes, ganz bevorzugte Formen zwischen 75 und 200 mg neben 5 bis 800 mg bzw. 5 bis 40 mg O-Acetylsalicylsäure. Im allgemeinen gibt man 2 bis 3 dieser Dosiseinheiten pro Tag, wobei je nach Schwere des Falles Abweichungen von dieser Dosierung nach oben und unten möglich sind. Die anzuwendende Dosierung hängt natürlich auch noch von anderen Faktoren ab, z.B. vom Alter, Gewicht, allgemeinen Gesundheitszustand des zu behandelnden Patienten, von der Schwere der Symptome bzw. der Erkrankung.

Die erfindungsgemäßen pharmazeutischen Zubereitungsformen enthalten gegebenenfalls noch sonstige übliche Träger- und/oder Hilfsstoffe und werden nach an sich üblichen Verfahren hergestellt. Als übliche Träger- und/oder Hilfsstoffe dienen z.B. Kartoffel-, Mais- oder Weizenstärke, Zellulose, Zellulosederivate, Siliziumdioxid, verschiedene Zucker, als Dragierstoffe Zucker und/oder Stärkesirup, Gelatine, Gummi arabicum, Polyvinylpyrrolidon, synthetische Zelluloseester, oberflächenaktive Substanzen, Weichmacher, und/oder Pigmente, zur Herstellung von Tabletten und Kerne auch Schmiermittel, wie Magnesiumstearat.

Zur Herstellung eines erfindungsgemäßen Dipyridamol-Granulats werden z.B. Dipyridamol mit einer organischen Genußsäure, wie Fumarsäure, Weinsäure, Zitronensäure, Bernsteinsäure, Äpfelsäure und mit Bindern und/oder Haftmitteln, wie z.B. Polyvinylpyrrolidon, gemischt, mit einem Schmiermittel, wie z.B. Magnesiumstearat versetzt, das Gemisch anschließend verdichtet, z.B. mit Hilfe eines Walzenkompaktors, und zu Granulaten aufgebrochen, z.B. unter Verwendung eines Trockengranuliergerätes mit nachgeschalteter Siebeinrichtung.

Die Herstellung von Dipyridamol-Pellets geschieht vorzugsweise unter Verwendung von Starterkernen, die vorteilhafterweise aus einer organischen Genußsäure, z.B. aus ausgerundeten Weinsäurekristallen, bestehen (Durchmesser der Starterkerne 0,5 bis 0,9 mm), auf welche in einem Kessel eine Suspension aus Dipyridamol in einem Alkohol oder Alkohol/-Wasser-Gemisch und aus einem Binder, wie z.B. Polyvinylpyrrolidon, so lange aufgesprüht wird, bis die entstehenden Wirkstoffpellets die vorgeschriebene Menge Dipyridamol enthalten (die Pellets weisen dann einen Durchmesser zwischen 0,9 und 1,5 mm auf). Diese Pellets werden, sofern man eine retardierte Wirkstoff-Abgabe beabsichtigt, mit einem Lack überzogen, der zu 50 bis 100% aus säureunlöslichen, darmsaftlöslichen Lacken und zu 50 bis 0% aus magen- und darmsaftunlöslichen Lacken besteht. Bewährt hat sich ein Lack aus Methacrylsäure-/Methacrylsäureester-Copolymeren (Eudragit S®) und Hydroxypropylmethylcellulosephthalat (HP 55®), dem noch Weichmacher und Füllstoffe, z.B. Talkum beigefügt sein können. Als darmsaftlösliche Lackkomponenten kommen auch noch Celluloseacetatphthalat, Ethylcellulosephthalat, Hydroxypropylmethylcellulosesuccinat, Celluloseacetatsuccinat, Hydroxypropylmethylcellulosehexahydrophthalat, Celluloseacetathexahydrophthalat, Hydroxypropylmethylcellulosetrimellitat, Methacrylsäure-Methacrylsäureester-Mischpolymerisat (Säurezahl 300 bis 330, Eudragit L®) oder Gemische dieser Stoffe in Frage. Darmsaft- und Magensaftunlösliche Lacke als Beimischungen können sein: Lacke auf Acrylat- bzw. Methacrylatbasis (Eudragit retard S® und Eudragit retard L®), auch in Verbindung von bis zu 14 Gew.-% Ethylcellulose.

Dipyridamol (und/oder Mopidamol) -Pellets lassen sich aber auch dadurch erhalten, daß man in einen Extruder (z.B. Doppelschneckenextruder) ein Pulvergemisch aus Dipyridamol (und/oder Mopidamol) und der berechneten Menge einer organischen Genußsäure (z.B. Fumarsäure) und Binder und gegebenenfalls sonstige Hilfsmittel einführt, hierzu noch ein organisches Lösungsmittel zudosiert und die Masse durchmischt. Die befeuchtete Masse wird in Form von Spraghettis ausgedrückt, die auf einer schnell drehenden Platte zu hochverdichteten Pellets gerundet werden. Die Pellets werden anschließend getrocknet und, gegebenenfalls, mit einem wie oben beschriebenen retardierenden Lack überzogen.

Die O-Acetylsalicylsäure kann einmal in Form von gepreßten Kernen, die mit einer isolierenden Deckschicht überzogen sind, oder in Form von Filmtabletten eingesetzt werden. Die Formlinge werden aus der O-Acetylsalicylsäure, Fließ- und Gleitmitteln und Träger- und/oder Verdünnungsmitteln, wie z.B. fließfähige Laktose, mikrokristalline Cellulose, getrocknete Maisstärke, Aluminium- oder Magnesiumstearat, in an sich bekannter Weise durch Herstellung und Verpreßung eines entsprechenden Granulats gewonnen. Die Kerne überzieht man, gegebenenfalls in mehreren Schritten, mit einer Dragiersuspension, die z.B. aus Zuckern (wie Saccharose), Gummi arabicum und Talk besteht.

Eine typische Drei-Schicht-Tablette wird beispielsweise dadurch gewonnen, daß ein Pyrimidopyrimidingranulat, ein O-Acetylsalicylsäure-Granulat und ein Trenngranulat, das z.B. aus Lactose, mikrokristalliner Zellulose und Polyvinylpyrrolidon aufgebaut ist und ein Gleitmittel enthält, mittels einer speziellen Tablettenpresse mit 3 Fülltrichtern und 3 Preßstation so verpreßt wird, daß die neutrale Trennschicht zwischen den beiden gepreßten Wirkstoffen liegt.

Die erfindungsgemäße Kombination wird als ein antithrombotisches, die Blutplättchenaggregation und die Metastasenwirkung inhibierendes Mittel bei Menschen und Tieren angewandt; die Kombination verhindert die Bildung und Aufrechterhaltung von venösen und arteriellen Blutgerinnseln, sie verhindert somit vorübergehende ischämische Anfälle und dient der vorbeugenden Beeinflussung zur Vermeidung von Herzinfarkten und Schlaganfällen. Sie ist gut geeignet zur Verhinderung der Bildung und Aufrechterhaltung eines Thrombus bei vorliegenden Arteriosklerosen oder nach operativen Eingriffen oder sonstigen mit Thromboseneigung einhergehenden Zuständen; bei der Anwendung kommen aber auch die typischen Eigenschaften der Einzelkomponenten zur Wirkung, z.B. kommt es zu einer verbesserten $O_2$-Versorgung des Herzmuskels und zur Hemmung entzündlicher Vorgänge, desweiteren zu einer Schmerzlinderung.

In-vivo Versuche an der Ratte:

Die Untersuchung der antithrombotischen Wirkung bei erfindungsgemäßer gleichzeitiger oraler Gabe von 5 mg/kg Dipyridamol und 0,05 mg/kg O-Acetylsalicylsäure (Gewichtsverhältnis Dipyridamol zu O-Acetylsalicylsäure=100) und, vergleichend hierzu, bei einer (gemäß der Europäischen Anmeldung Nr. 69465) zeitlich versetzt verabreichten oralen Gabe von 5 mg/kg Dipyridamol und 5 mg/kg O-Acetylsalicylsäure an Ratten (FW 49) mit einem Körpergewicht von 60 bis 80 g vor Beginn der Untersuchung, erfolgte durch das Setzen eines Reizes zur Thrombusbildung an Gefäßen des Mesenteriums und durch die Beobachtung des zeitlichen Verhaltens der Größe dieser Thromben. Es wurden jeweils Gruppen von 5 Tieren verwendet. Ein standardisierter Reiz führt zu einem Thrombus mit einer Größe, die 80% des Gefäßdurchmessers verschließt. Zwischen der gleichzeitigen oralen Gabe der Substanzen gemäß vorliegender Erfindung und der Setzung des Thrombus bzw. der eigentlichen Durchführung der Messungen wird ein Intervall von einer Stunde gewählt. Die zeitlich versetzte orale Gabe der Substanzen gemäß der obengennanten europäischen Anmeldung erfolgte so, daß zuerst Dipyridamol 90 Minuten vor der Gabe der O-Acetylsalicylsäure, letztere 60 Minuten vor dem Setzen des Thrombus und den beginnenden Messungen erfolgte; mit anderen Worten, Dipyridamol wurde 150 Minuten, O-Acetylsalicylsäure 60 Minuten vor Versuchsbeginn gegeben.

Zur Durchführung des Versuches wurden die Tiere mit ca. 60 mg/kg Nembutal i.p. narkotisiert, ihr Abdomen eröffnet und ein Teil des Mesenteriums nach außen luxiert und mit physiologischer, erwärmter Nährlösung während des Ablaufs der Messungen überspült.

Unter intravitalmikroskopischer Beobachtung wurde eine Platinelektrode auf eine äußere Gefäßwand des Mesenteriums gesetzt. Eine Gegenelektrode wurde unter das Mesenterium geschoben. Eine fest gewählte Kombination zwischen angelegter Gleichspannung (150 V) und Strom (1,5 mA) in einem Impuls mit vorgegebener Länge (100 ms) führt bei einer Venole von ca. 300 µm Durchmesser zu einer reproduzierbaren Bildung eines Thrombus, der in aller Regel 80% des Gefäßdurchmessers verschließt. Die Größe des Thrombus wird im Abstand von 10 Sekunden, später von 30 Sekunden in einem Zeitbereich bis zu 20 Minuten nach Reizung des Gefäßes gemessen und als Thrombusgröße (senkrecht zur Gefäßwand) in Prozent des Gefäßinnendurchmessers angegeben.

Innerhalb des Beobachtungsintervalles von 20 Minuten ist bei dem jeweiligen Kontrollkollektiv, das aus nur mit dem jeweiligen Lösungs- bzw. Trägermittel oral behandelten Tieren besteht, eine stabile Thrombusgröße von ca. 85% zu beobachten (vgl. Abbildung 7, N=68 Kontrollmessungen; Mittelwerte zu jedem Zeitpunkt mit Angabe der Standardabweichung [± SD]).

Für akut auftretende Gefäßverschlüsse, wie z.B. bei einem Herzinfarkt oder bei einem Schlaganfall, wird allgemein ein rasch gebildeter Thrombus an einer bestehenden Verletzung oder krankhaften Veränderung der Gefäßwand verantwortlich gemacht. Bei einer die Thrombusbildung hemmenden Medikation ist zu erwarten, daß der entstehende Thrombus die in der Kontrollgruppe erreichte Größe von 85% des Gefäßdurchmessers nicht erreicht. Nur so kann verhindert werden, daß es durch die ungehemmte Bildung eines Thrombus zu einer Verlegung des Blutflusses kommt, was üblicherweise schwer wiegende Folgen durch die dann entstehenden ischämischen Schäden nach sich zieht (z.B. Herzinfarkt, Schlaganfall). Eine Medikation mit einer Substanz oder Substanzkombination, die nur eine schnelle Auflösung eines Thrombus zur Folge hat, wird im allgemeinen die Bildung des Thrombus bis zu einer mit der Kontrollgruppe vergleichbaren Größe zulassen, um diesen jedoch in dem nachfolgenden Untersuchungszeitraum deutlich zu verringern, d.h. eine initiale Verlegung des Blutflusses wird somit nicht verhindert. Die Abbildungen 1 und 2 zeigen den zeitlichen Verlauf der Thrombusgröße nach elektrischer Thrombussetzung für eine Kombination gemäß der oben angegebenen Europäischen Patentanmeldung EP—A—69465 (Abbildung 1) und eine erfindungsgemäße Kombination (Abbildung 2).

Die Abbildung 1 zeigt den Verlauf der Thrombusgröße innerhalb von 20 Minuten nach Setzen des Thrombus nach einer oralen Gabe von 5 mg/kg O-Acetylsalicylsäure 60 Minuten vor Durchführung der Messung und einer oralen Gabe von 5 mg/kg Dipyridamol 150 Minuten vor Durchführung der Messung. Bei der Kontrollgruppe bleibt dabei die Größe des Thrombus während der ersten Hälfte des Beobachtungszeitraumes bei ca. 85%, um in der zweiten Hälfte geringfügig abzufallen, während in der behandelten Gruppe die Größe des Thrombus zunächst innerhalb der ersten 30 Sekunden Werte nahe derjenigen des Kontrollversuchs annimmt, um im weiteren Versuchsverlauf jedoch sich stärker zu verringern.

Ein deutlich unterschiedlicher Zeitverlauf zeigt sich in Abbildung 2. Darin ist eine Kontrollgruppe mit einer Gruppe verglichen worden, die erfindungsgemäß gleichzeitig oral 5 mg/kg Dipyridamol und 0,05 mg/kg O-Acetylsalicylsäure 60 Minuten vor dem Beginn der Messungen erhalten hat. Im Gegensatz zu der Abbildung 1 zeigt die behandelte Gruppe bereits zu Beginn und im ersten Zeitabschnitt des Versuches eine deutlich gehemmte Bildung des Thrombus, die bereits zu Beginn des Versuchs die Werte annimmt, die im Falle der behandelten Gruppe der Abbildung 1 erst im zweiten Drittel des Versuches erreicht wird.

Aus diesen Zeitverläufen der Abbildungen 1 und 2 wird erkennbar, daß die der Abbildung 2 zugrundegelegte Vorbehandlung bereits die Entstehung des Thrombus verhinderte, während die der Abbildung 1 zugrundeliegende Behandlung lediglich eine beschleunigte Auflösung des gesetzten Thrombus bewirkte, die aber auch bei der Behandlung, die der Abbildung 2 zugrunde liegt, in gleichem Maße zu beobachten ist.

In den Abbildungen 3 bis 6 werden die prozentualen Thrombusgrößen in einzelnen Zeitabschnitten zusammengefaßt und jeweils einander gegenübergestellt. Die Kolonnen A repräsentieren die Thrombusgrößen der Kontrollgruppe, d.h. die Thrombusgröße der Kontrollgruppe ist auf 100% gesetzt worden. Die Kolonnen B zeigen die Thrombusgrößen einer Kombination von 5 mg/kg Dipyridamol und 5 mg/kg O-Acetylsalicylsäure entsprechend der Kurve in Abbildung 1, die Kolonnen C die Thrombusgrößen bei vorheriger Applikation von 2,5 mg/kg O-Acetylsalicylsäure, die Kolonnen D bei vorheriger Applikation von 5 mg/kg Dipyridamol und die Kolonnen E nach vorheriger Applikation der erfindungsgemäßen Kombination von 5 mg/kg Dipyridamol und 0,05 mg/kg O-Acetylsalicylsäure entsprechend der Kurve in Abbildung 2, die Kolonnen F von 2 mg/kg Dipyridamol und 0,05 mg/kg O-Acetylsalicylsäure (alle Applikationen erfolgten oral).

Die Abbildung 3 gibt die prozentuale Reduktion des 85%igen Verschlußes in der Zeit von 10 Sekunden bis 1 Minute nach der Reizung des Gefäßes wieder, verglichen mit den Kontrollen, die mit 100% eingesetzt sind. Wie man hieraus sieht, ist bei Vorhandensein der erfindungsgemäßen Kombination (Kolonne E) die Größe des bei der elektrischen Reizung gesetzten Thrombus bereits um ca. 20% (also praktisch schon in statu nascendi) verringert; nach der Verabreichung der vorbekannten Kombination (Kolonne B) dagegen nur um ca. 5%; dies bedeutet, daß bei Verabreichung der erfindungsgemäßen Kombination die Thrombusbildung bereits deutlich gehindert wird.

Die Abbildung 4 zeigt die gleichen Verhältnisse im Zeitbereich 2 bis 4 Minuten ab der Reizung. Die Thrombusgröße nimmt allgemein ab, am stärksten ausgeprägt ist die Reduktion bei der erfindungsgemäßen Kombination.

Die Abbildung 5 gibt die Verhältnisse im Zeitbereich 5 bis 10 Minuten nach Reizung, die Abbildung den Zeitbereich 10 bis 20 Minuten wieder. Erst nach ca. 15 Minuten wird auch bei der vorbekannten Kombination eine Reduktion der Thrombusgröße erreicht, welche der durch die erfindungsgemäße Kombination erzielten entspricht. Die O-Acetylsalicylsäure und das Dipyridamol allein bewirken keine signifikante Verminderung der Thrombusgröße. Vergleicht man besonders in Abbildung 6 die prozentualen Senkungen miteinander, so sieht man, daß es sich bei den Acetylsalicylsäure-Dipyridamol-Kombinationen um überadditive bzw. synergistische Effekte handelt. Aus der Abbildung 3 wird aber noch ein weiterer Zusammenhang sichtbar: bei Erhöhung der O-Acetylsalicylsäuredosis gegenüber der Dipyridamoldosis nimmt der hemmende Effekt auf die Thrombusbildung ab; es hat sich gezeigt, daß bei Kombinationen mit Gewichtsverhältnissen von Dipyridamol zu O-Acetylsalicylsäure von kleiner als 4 sehr schnell Verhältnisse erzielt werden, die der in der Abbildung 1 gezeigten Kurve bzw. den in den Abbildungen 3 bis 6 durch die Kolonnen B gezeigten Werten entsprechen. Dadurch wird aber auch klar ersichtlich, daß die erfindungsgemäßen Kombinationen den vorbekannten Kombinationen bei der Thrombusverhütung deutlich überlegen sind. Dabei sollte nicht unerwähnten bleiben, daß dieser Effekt mit einer Kombination erreicht wird, in der der O-Acetylsalicylsäureanteil sehr gering ist, so daß die Nebenwirkungen dieser Substanz nicht zu erwarten sind.

Dafür, daß nicht nur die Applikationsfolge von Dipyridamol und Acetylsalicylsäure sondern bei gleichzeitiger Applikation auch die Dosisrelation dieser beiden Wirkstoffe von entscheidender Bedeutung für die Verhinderung von Gefäßverschlüssen durch Thromben ist, zeigten Versuche am Modell der rekurrierenden Thrombose an der Kaninchenaorta. In Anlehnung an das von Folts, JD, in Circulation 54 (1976), Seite 365, beschriebene Modell an der Arteria circumflexa des Hundes wurde ein rekurrierndes Modell an der abdominalen Aorta des Kaninchens entwickelt. Nach Anaesthesie mit Rompun, initial mit 10 mg/kg Rompun und 70 mg/kg Ketanest i.m. und, kontinuierlich, mit 4 mg/kg/h Rompun und 20 mg/kg/h Ketanest i.m. während der Dauer des Versuches (Dauerinfusion), wurden Kaninchen (New Zealand Whites von ca. 3 kg Körpergewicht) durch Sagitalschnitt laparotomiert. Die abdominale Aorta wurde distal von der Arteria renalis freigelegt; es wurde eine elektromagnetische Flow-Sonde (alternativ eignet sich hierzu auch eine Ultraschall-Flow-Sonde) am proximalen Ende dieses Abschnittes angebracht. Distal zu der Flow-Sonde wurde die Aorta mechanisch durch wiederholtes Quetschen mittels einer Arterienklemme (eines Hemostats) geschädigt. Diese Prozedur führte zu einer Gefäßschädigung durch Ruptur der Lumenauskleidung, Freilegung subendothelialer Strukturen, Bindegewebe und glatter Muskeln. Am Orte der Gefäßschädigung wird eine mechanische Stenose angebracht, die den Fluß durch die Aorta auf ungefähr 40% des Ausgangswertes reduziert.

Nach dieser Schädigung beobachtet man, daß der totale Volumenfluß durch die Aorta im Verlauf von einigen Minuten stetig reduziert wird bis zum vollständigen Sistieren des Flusses. Das Gefäß bleibt verschlossen, bis der gebildete Thrombus in dem verengten Segment durch mechanische Agitation losgelöst, d.h. embolisiert wird. Dadurch wird der ursprüngliche Fluß wieder hergestellt. Danach folgt wiederum eine graduelle Abnahme des Blutflusses bis zum erneuten Stillstand. Nach erneuter mechanischer Agitation bzw. Embolisierung des Thrombus läßt sich dieser rekurrierende Prozeß immer und immer wieder verfolgen.

Zu Beginn eines jeden Versuchs wird eine Anzahl von Flow-Reduktions-Cyclen über eine Periode von 30 Minuten zu Kontrollzwecken festgehalten. Darauf wird der zu prüfende Wirkstoff verabreicht, etnweder intravenös oder in die Mesenterialvene. Über eine Periode von 30 bis 60 Minuten sowie über eine weitere Periode von 60 bis 90 Minuten nach der Applikation des Wirkstoffes werden die beobachteten Flow-Reduktions-Cyclen festgehalten. Ein wirksamer Stoff verzögert das Versiegen des Blutflusses, embolisiert spontan den sich bildenden Thrombus in dem stenosierten Segment oder stellt—bei guter Wirkung—den ursprünglichen Fluß des Blutes ohne periodische Reduktion wieder her. Der maximal erreichbare antithrombotische Effekt entspricht also der vollständigen Wiederherstellung des durch die Stenose möglichen maximalen Blutflusses (100% "free Flow").

Für die numerische Auswertung der Ergebnisse wird das Muster der Flow-Reduktions-Zyclen vor Gabe des Wirkstoffes zu Vergleichszwecken ermittelt. Aus dem Unterschied in der Flow-Reduktions-Rate und der Amplitude der zyklischen Flow-Oscilation wird mit einem Computer ein Flow-Muster konstruiert, das die Zunahme des freien Flusses zu jedem Zeitpunkt nach der Wirkstoffgabe wiedergibt. Mechanisch herbeigeführte Thrombus-Embolisierungen werden negativ veranschlagt im Vergleich zu spontanen Thrombus-Embolisierungen (d.h. es erfolgt keine oder, gegebenenfalls eine positive Korrektur). Aus diesen korrigiereten Kurven errechnet der Computer den Parameter "Freier Fluß" ("free Flow") für 20 oder 30 minütige Intervalle vor und nach der Wirkstoffgabe. Um topische Effekte, die bei oraler Gabe der Acetylsalicylsäure auftreten können, zu minimieren, wurden die Wirkstoffe als Bolus in die Mesenterialvene verabreicht.

Ergebnisse:

100 %iger freier Flow bedeutet einen ungehemmten Fluß durch die Aorta ohne Hemmung durch Thromben (jedoch mit besagter mechanischer Einengung). Es wurden jeweils vier Tiere bei jeder Gruppe verwendet. Es wurden folgende Dosen bzw. Dosis-Kombinationen angewandt:

| | |
|---|---|
| 1.) Dipyridamol | 5 mg/kg |
| 2.) Acetylsalicylsäure | 50 µg/kg |
| 3.) Acetylsalicylsäure | 100 µg/kg |
| 4.) Acetylsalicylsäure | 1 mg/kg |
| 5.) Acetylsalicylsäure | 500 µg/kg |
| 6.) Acetylsalicylsäure+Dipyridamol | 500 µg/kg+5 mg/kg (Abb. 8) |
| 7.) Acetylsalicylsäure+Dipyridamol | 50 µg/kg+5 mg/kg (Abb. 9) |
| 8.) Acetylsalicylsäure+Dipyridamol | 100 µg/kg+5 mg/kg (Abb. 10) |
| 9.) Acetylsalicylsäure+Dipyridamol | 5 mg/kg+1 mg/kg (Abb. 11) |

Die ersten fünft Einzelsubstanz-Behandlungen zeigten überhaupt keine Wirkungen in diesem Modell. Kombinationen von Dipyridamol mit Acetylsalicylsäure, die simultan gegeben wurden, zeigten dagegen eine Zunahme des freien Flußes mit zunehmender Zeit. Hierzu wird auf die Abbildungen 8 bis 10 verwiesen. Aus diesen Figuren wird ersichtlich, daß das Verhältnis von Dipyridamol zu Acetylsalicylsäure von 10:1 weniger wirksam ist als das von 100:1, letzteres ist aber wiederum weniger wirksam als das von 50:1, was die Zunahme des freien Flusses betrifft.

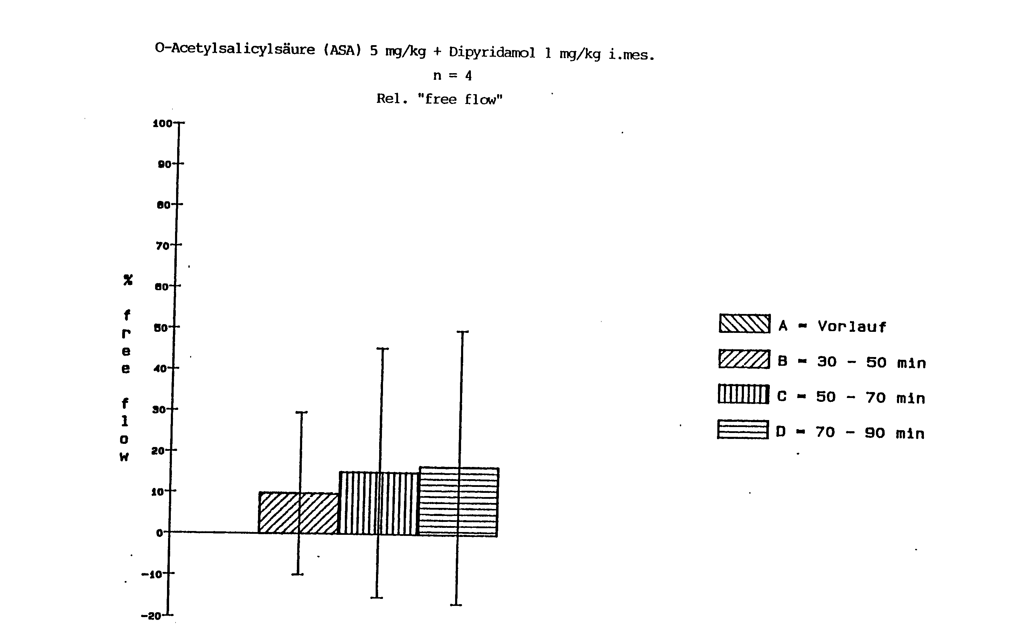

Die Abbildung 11 gibt in etwa die Verhältnisse bei einer bekannten Kombination (Asasantin®) wieder; es wird ersichtlich, daß diese deutlich weniger wirksam ist als die erfindungsgemäßen Kombinationen. Da bei den bischerigen bekannten Kombinationen stets vergleichsweise hohe Konzentrationen an Acetylsalicylsäure verwendet werden, ist auch bisher auf ein erhöhtes Blutungsrisiko wiederholt hingewiesen worden. Die erfindungsgemäßen Kombinationen zeigen überraschenderweise eine deutlich bessere Wirkung bei einer wesentlich verringerten Konzentration von Acetylsalicylsäure, was eine sehr deutliche Verringerung bzw. sogar Vermeidung des Blutungsrisikos bedeutet.

Eine Kombination der beiden Wirkstoffe interveniert bereits im ersten Stadium der Thrombus-Bildung; die Gefahr einer rekurriernden Thrombosis in einen System mit hohem Druck und hohen Fluß- und Scherraten ("high flow/high pressure"—System) wird signifikant vermindert. Die bevorzugte Dosis liegt (beim Kaninchen) bei 50:1 (Dipyridamol zu Acetylsalicylsäure). Man sollte aber nicht außer Acht lassen, daß Kaninchen im allgemeinen weniger sensitiv auf eine antithrombotische Behandlung ansprechen als andere Tiere oder der Mensch. Aus den Versuchen ist zu entnehmen, daß eine Kombination von Dipyridamol und Acetalsalicylsäure die arterielle Thrombus-Bildung wesentlich wirksamer verhindert als eine Monotherapie. Die Überlegenheit der Behandlung mit einem Verhältnis von 100:1 gegenüber der mit einem Verhältnis von 10:1 bezüglich der Verhinderung der Thrombusbildung bestätigt die weiter oben genannten Ergebnisse bei Versuchen mit dem Mesenterialvenenmodell der Ratte.

Die folgenden Beispiele sollen die Erfindung nähers erläutern:

Beispiel 1

Kapseln enthaltend Dipyridamol-Depot-Formen neben einem Dragée mit O-Acetylsalicylsäure

a.) Dipyridamol-Pellets mit verzögerter Wirkskstoff-Freigabe

300 kg ausgerundete Weinsäure-Starterpellets werden in einem Spezialkessel mit einer Suspension bestehend aus Isopropanol, Dipyridamol und Polyvinylpyrrolidon solange besprüht, bis die entstehenden Wirkstoffpellets ca. 45% Dipyridamol enthalten.

Diese Pellets besprüht man mit einem Lack, der aus Methacrylsäure/Methylmethacrylat-Copolymer (Handelsname Eudragit S) und Hydroxypropylmethylcellulosephthalat (Handelsname HP 55) im Gewichtsverhältnis 85:15 bis 50:50 besteht. Die organischen Lacklösungen enthalten noch Weichmacher und Talkum. Es werden zwei Pelletkomponenten mit 5 und 7% Hüllenanteil und unterschiedlichem Verhältnis der Lackkomponenten in den genannten Grenzen gesprüht. Die beiden Komponenten werden so gemischt, daß sie nachfolgende in vitro Freigabe ergeben:

Bedingungen: entsprechend US P XXI, Basket-Methode, 100 Umdrehungen/Min.
1 Stunde künstlicher Magensaft,
2 bis 6 Stunden künstlicher Darmsaft (Phosphatpuffer pH 5,5)

| Zeit | Prozentuale Wirkstoff-Freigabe pro Stunde |
|---|---|
| 1. Stunde | ca. 30% |
| 2. Stunde | ca. 25% |
| 3. Stunde | ca. 18% |
| 4. Stunde | ca. 12% |

nach der 6. Stunde mehr als 90% Dipyridamol-Freigabe.

b.) O-Acetylsalicylsäure enthaltende Dragées

Wie im Beispiel 1 beschrieben werden 100 mg schwere Acetylsalicylsäure-Kerne durch Verpressung folgender Mischung hergestellt:

| | |
|---|---|
| O-Acetylsalicylsäure | 25,0 Gew.-% |
| Lactose | 53,0 Gew.-% |
| Mikrokristalline Cellulose | 11,0 Gew.-% |
| Maisstärke, getrocknet | 8,6 Gew.-% |
| Siliciumdioxid | 3,0 Gew.-% |
| Aluminiumstearat | 0,4 Gew.-% |

Diese Kerne werden wie beschrieben solange mit der genannten Dragiersuspension überzogen, bis sie nach guter Trocknung 120 mg wiegen.

c.) Abfüllung:

Auf einer Spezial-Kapselmaschine werden in eine Kapsel, Größe O, die 200 mg Dipyridamol entsprechende Pellet-Menge und ein 25 mg Acetylsalicylsäure enthaltendes Dragée eingefüllt.

Das Gewichtsverhältnis Dipyridamol zu O-Acetylsalicylsäure beträgt 8.

Beispiel 2

Kapseln enthaltend eine Dipyridamol-Depot-Form neben einem Dragée mit O-Acetylsalicylsäure

a.) Dipyridamol-Pellets mit verzögerter Wirkstoff-Freigabe

In einem Doppelschneckenextruder werden ein Dipyridamol enthaltendes Pulvergemisch und ein organisches Lösungsmittel im richtigen Verhältnis hineindosiert und miteinander vermischt. Die befeuchtete Masse wird in Form von Spaghettis ausgedrückt, die in einem Behälter mit einer schnell drehenden Bodenplatte zu hoch verdichteten Pellets gerundet werden. Die Pellets trocknet man anschließend in einem Trockenschrank.

Pulverzusammensetzung:

| | |
|---|---|
| Dipyridamol | 69,0 Gew.-% |
| Äthylcellulose | 5,5 Gew.-% |
| Hydroxypropylmethylcellulose, hochpolymer | 12,5 Gew.-% |
| Polyäthylenglykol 6000 | 1,0 Gew.-% |
| Fumarsäure | 12,0 Gew.-% |

b.) Acetylsalicylsäure-Dragées

Wie in den Beispielen 1 und 2 beschrieben stellt man unter Verwendung der gleichen Hilfsstoffe ein 75 mg schweres Acetylsalicylsäure-Dragée her, das 5 mg dieses Stoffes enthält.

c.) Abfüllung:

Auf einer Spezial-Kapselmaschine werden in eine Kapsel der Größe 00 eine Pelletmenge, die 450 mg Dipyridamol entspricht, und ein 5 mg Acetylsalicylsäure enthaltendes Dragée eingefüllt.

Das Gewichtsverhältnis Dipyridamol zu O-Acetylsalicylsäure beträgt 90.

Beispiel 3

Kapseln als Instant-Form enthaltend Dipyridamol und O-Acetylsalicylsäure

Herstellung:

a.) Dipyridamol-Granulat:

Eine Mischung aus 30% Dipyridamol, 63% Fumarsäure und 6% Polyvinylpyrrolidon wird mit Äthanol befeuchtet und durch ein Sieb mit einer Maschenweite von 1,5 mm gesiebt. Nach Trocknung mischt man 1% Magnesiumstearat zu und verdichtet das Granulat auf einem Walzenkompaktor, der mit einem Trockengranuliergerät mit Siebeinrichtung ausgestattet ist.

Die Fraktion der Korngröße von 0,4 bis 1,25 mm wird weiterverwendet (prozentuale Angaben in Gew.-%).

b.) Abfüllung:

Auf einer Spezial-Kapselmaschine werden in eine Kapsel, Größe 0, die 100 mg Dipyridamol entsprechende Menge an Granulat und ein 5 mg Acetylsalicylsäure enthaltendes Dragée entsprechend Beispiel 3 eingefüllt.

Die Wirkstofffreigabe des Dipyridamol-enthaltenden Granulates ist pH-unabhängig:

| pH-Wert künstlicher Magen- bzw. Darmsaft | in vitro-Dipyridamol- in Minuten* | |
|---|---|---|
| | 50% | >90% |
| 1,2 | 3 | 11 |
| 4,0 | 4 | 13 |
| 6,0 | 4 | 15 |

*US P XXI—Paddle—Methode
100 Umdrehungen/Min.

Das Gewichtsverhältnis von Dipyridamol zu O-Acetylsalicylsäure beträgt 20.

**Patentansprüche für die Vertragsstaaten: BE, CH, DE, FR, GB, IT, LI, LU, NL, SE**

1. Arzneimittelkombination enthaltend Dipyridamol und/oder Mopidamol bzw. deren physiologisch unbedenkliche Salze und O-Acetylsalicylsäure bzw. ihre physiologisch unbedenkliche Salze, wobei beide Komponenten gegeneinander isoliert vorliegen, dadurch gekennzeichnet, daß die Pyrimidopyrimidin-Komponente und die O-Acetylsalicylsäure-Komponente in einem Gewichtsverhältnis von 8:1 und größer in Verbindung mit oder ohne pharmazeutische Träger vorliegen.

2. Arzneimittelkombination gemäß Anspruch 1, dadurch gekennzeichnet, daß diese die Pyrimidopyrimidin-Komponente und die O-Acetylsalicylsäure-Komponente in einem Gewichtsverhältnis von 8:1 bis zu 100:1 enthält.

3. Arzneimittelkombination gemäß Anspruch 1 und 2, dadurch gekennzeichnet, daß Dipyridamol und/oder dessen pharmakologisch verträgliche Salze als Granulate neben einem oder mehreren, die O-Acetylsalicylsäure oder ihre physiologisch verträglichen Salze enthaltenden Dragées vorliegen und, gegebenenfalls, beide Formen in einer Kapsel abgefüllt sind.

4. Arzneimittelkombination gemäß Anspruch 1 und 2, dadurch gekennzeichnet, daß Dipyridamol und/oder dessen physiologisch verträgliche Salze zusammen mit einem sauren Hilfsstoff als Pellets in einem Verhältnis von mindestens 1 Val des sauren Hilfsstoffes zu 1 Mol Dipyridamol vorliegen, wobei diese Pellets mit einem magensaftunlöslichen, darmsaftlöslichen Lack, der im Magen-Darm-Trakt eine saure Lösung dieses Wirkstoffes gleichmäßig retardiert frei gibt, umgeben sind, neben einem oder mehreren, die O-Acetylsalicylsäure oder ihre physiologisch verträglichen Salze enthaltenden Dragées und, gegebenenfalls, beide Formen in einer Kapsel abgefüllt sind.

5. Arzneimittelkombination gemäß Anspruch 1 bis 5, dadurch gekennzeichnet, daß diese zwischen 10 und 675 mg Dipyridamol und/oder Mopidamol und 1 und 150 mg O-Acetylsalicylsäure oder die physiologisch verträglichen Salze dieser Wirkstoffe, vorzugsweise aber zwischen 75 und 200 mg Dipyridamol und/oder Mopidamol und 5 bis 80 mg O-Acetylsalicylsäure oder die physiologisch verträglichen Salze dieser Wirkstoffe enthalten.

6. Verfahren zur Herstellung von Arzneimittelkombinationen enthaltend Dipyridamol und/oder Mopidamol bzw. deren physiologisch unbedenkliche Salze und O-Acetylsalicylsäure bzw. deren physiologisch unbedenkliche Salze, wobei mindestens eine dieser beiden Komponenten gegenüber der anderen durch eine Trennschicht geschützt ist, dadurch gekennzeichnet, daß die Pyrimidopyrimidin-Komponente und die O-Acetylsalicylsäure-Komponente im Gewichtsverhältnis von 8:1 und größer zusammengemischt, gegebenenfalls beide Komponenten vorher mit an sich üblichen Träger und/oder Hilfsmitteln versehen werden, und die eine oder beide dieser Komponenten in Form von Granulaten, Pellets oder Dragées zugemischt werden, und mindestens eine dieser Komponenten mit einer Umhüllung versehen wird, die eine gleichzeitige Abgabe der Wirkstoffe in den Magen-Darm-Trakt gewährleistet.

7. Verwendung einer Arzneimittelkombination gemäß Anspruch 1 zur Herstellung eines Arzneimittels mit einer die Bildung von Thromben inhibierenden Wirkung.

**Patentansprüche für die Vertragsstaaten: AT, ES, GR**

1. Verfahren zur Herstellung von Arzneimittelkombinationen enthaltend Dipyridamol und/oder Mopidamol bzw. deren physiologisch unbedenkliche Salze und O-Acetylsalicylsäure bzw. deren physiologisch unbedenkliche Salze, wobei mindestens eine dieser beiden Komponenten gegenüber der anderen durch eine Trennschicht geschützt wird, dadurch gekennzeichnet, daß die Pyrimidopyrimidin-Komponente und die O-Acetylsalicylsäure-Komponente im Gewichtsverhältnis von 8:1 und größer zusammengemischt, gegebenenfalls beide Komponenten vorher mit an sich üblichen Träger und/oder Hilfsmitteln versehen werden, und die eine oder beide dieser Komponenten in Form von Granulaten, Pellets oder Dragées zugemischt werden, und mindestens eine dieser Komponenten mit einer Umhüllung versehen wird, die eine gleichzeitige Abgabe der Wirkstoffe in den Magen-Darm-Trakt gewährleistet.

2. Verfahren gemäß Anspruch 1 zur Herstellung einer Instant-Form, dadurch gekennzeichnet, daß Dipyridamol- oder Mopidamol-Pellets oder -Granulate mit einem magensaftunlöslichen, darmsaftlöslichen Lack überzogen werden und diese Pellets oder Granulate mit der O-Acetylsalicylsäure vermischt werden.

3. Verfahren gemäß Anspruch 1 zur Herstellung von Instant-Formen, dadurch gekennzeichnet, daß O-Acetylsalicylsäure-Kerne oder -Tabletten mit einem essigsäuredichten Überzug, der im Magensaft schnell lösbar ist, versehen und diese anschließend mit Dipyridamol- oder Mopidamol-Granulaten oder -Pellets vermischt werden.

4. Verfahren gemäß Anspruch 1 und 3, dadurch gekennzeichnet, daß ein Dipyridamol- oder Mopidamol-Granulat oder -Pellets zusammen mit einem oder mehreren eine Schutzschicht tragenden Dragées oder Filmtabletten, enthaltend die O-Acetylsalicylsäure, in Kapseln abgefüllt werden.

**Revendications pour les Etats Contractants: BE, CH, DE, FR, GB, IT, LI, LU, NL, SE**

1. Combinaison médicamenteuse contenant du dipyridamole et/ou du mopidamole ou respectivement leurs sels physiologiquement inoffensifs et de l'acide O-acétylsalicylique ou respectivement ses sels physiologiquement inoffensifs, dans laquelle les deux composants se présentent isolés l'un par rapport à l'autre, caractérisée en ce que le composant de type pyrimidopyrimidine et le composant acide O-acétylsalicylique se présentent dans un rapport en poids de 8:1 et plus, en combinaison avec ou sans support pharmaceutique.

2. Combinaison médicamenteuse selon la revendication 1, caractérisée en ce que celle-ci contient le composant pyrimidopyrimidine et le composant acide O-acétylsalicylique dans un rapport en poids de 8:1 à 100:1.

3. Combinaison médicamenteuse selon la revendication 1 et 2, caractérisée en ce que le dipyridamole et/ou ses sels pharmacologiquement supportables se présentent sous forme de granulés conjointement à une ou plusieurs dragées contenant l'acide O-acétylsalicylique ou ses sels physiologiquement supportables et, éventuellement, les deux formes sont introduites dans une capsule.

4. Combinaison médicamenteuse selon la revendication 1 et 2, caractérisée en ce que le dipyridamole et/ou ses sels physiologiquement supportables, ensemble avec un adjuvant acide, se présentent sous forme de boulettes dans un rapport d'au moins 1 val de l'adjuvant acide à 1 mol de dipyridamole, ces boulettes étant enrobées d'un vernis soluble dans le suc intestinal, insoluble dans le suc gastrique, vernis qui libère de façon retardée et régulière une solution acide de cette substance active dans le tractus gastro-intestinal, conjointement à une ou plusieurs dragées contenant l'acide O-acétylsalicylique ou ses sels physiologiquement supportables et, éventuellement, les deux formes sont introduites dans une capsule.

5. Combinaison médicamenteuse selon la revendication 1 à 5, caractérisée en ce que celle-ci contient entre 10 et 675 mg de dipyridamole et/ou de mopidamole et entre 1 et 150 mg d'acide O-acétylsalicylique ou les sels physiologiquement supportables de ces substances actives, de préférence toutefois entre 75 et 200 mg de dipyridamole et/ou de mopidamole et 5 à 80 mg d'acide O-acétylsalicylique ou les sels physiologiquement supportables de ces substances actives.

6. Procédé pour la fabrication de combinaisons médicamenteuses contenant du dipyridamole et/ou du mopidamole ou respectivement leurs sels physiologiquement inoffensifs et de l'acide O-acétylsalicyclique ou respectivement ses sels physiologiquement inoffensifs, dans lequel l'un au moins de ces deux composants est protégé vis-à-vis de l'autre par une couche séparatrice, caractérisé en ce que le composant pyrimidopyrimidine et le composant acide O-acétylsalicylique sont mélangés ensemble dans le rapport en poids de 8:1 et plus, éventuellement les deux composants étant au préalable munis d'excipients et/ou d'adjuvants usuels, et en ce que l'un ou les deux de ces composants sont ajoutés en mélange sous forme de granulés, de boulettes, ou de dragées, et au moins l'un de ces composants est muni d'un enrobage qui assure une libération simultanée des substances actives dans le tractus gastro-intestinal.

7. Utilisation d'une combinaison médicamenteuse selon la revendication 1 pour la fabrication d'un médicament présentant une activité inhibant la formation de thromboses.

**Revendications pour les Etats Contractants: AT, ES, GR**

1. Procédé pour la fabrication de combinaisons médicamenteuses contenant du dipyridamole et/ou du mopidamole ou respectivement leurs sels physiologiquement inoffensifs et de l'acide O-acétylsalicylique ou respectivement ses sels physiologiquement inoffensifs, dans lequel au moins l'un de ces deux composants est protégé vis-à-vis de l'autre par une couche séparatrice, caractérisé en ce que le composant pyrimidopyrimidine et le composant acide O-acétylsalicylique sont mélangés ensemble dans le rapport en poids de 8:1 et plus, éventuellement les deux composants étant au préalable munis d'excipients et/ou d'adjuvants usuels en soi, et en ce que l'un ou les deux de ces composants sont ajoutés en mélange sous forme de granulés, de boulettes, ou de dragées, et au moins l'un de ces composants est muni d'un enrobage qui assure une libération simultanée des matières actives dans le tractus gastro-intestinal.

2. Procédé selon la revendication 1 pour la fabrication d'une forme instantanée, caractérisé en ce que les boulettes ou granulés de dipyridamole ou de mopidamole sont enrobés d'un vernis soluble dans le suc intestinal, insoluble dans le suc gastrique et ces boulettes ou granulés sont mélangés avec l'acide O-acétylsalicylique.

3. Procédé selon la revendication 1 pour la fabrication de formes instantanées, caractérisé en ce que des noyaux ou comprimés d'acide O-acétylsalicylique sont munis d'un enrobage résistant à l'acide acétique, lequel est rapidement soluble dans le suc gastrique et en ce que ceux-ci sont ensuite mélangés avec des granulés ou des boulettes de dipyridamole ou de mopidamole.

4. Procédé selon la revendication 1 et 3, caractérisé en ce que des granulés ou des boulettes de dipyridamole ou de mopidamole, ensemble avec une ou plusieurs dragées ou un ou plusieurs comprimés enrobé(e)s portant une couche protectrice, contenant l'acide O-acétylsalicylique, sont introduits dans des capsules.

**Claims for the Contracting States: BE, CH, DE, FR, GB, IT, LI, LU, NL, SE**

1. Pharmaceutical combination containing dipyridamole and/or mopidamol or the physiologically acceptable salts thereof and O-acetylsalicylic acid or the physiologically acceptable salts thereof, the two components being isolated from each other, characterised in that the pyrimido-pyrimidine component and the O-acetylsalicylic acid component are present in a weight ratio of 8:1 or more, either with or without pharmaceutical carriers.

2. Pharmaceutical combination as claimed in claims 1 and 2, characterised in that it contains the pyrimido-pyrimidine component and the O-acetylsalicylic acid component in a weight ratio of 8:1 up to 100:1.

3. Pharmaceutical combination as claimed in claims 1 and 2, characterised in that dipyridamole and/or the physiologically acceptable salts thereof are present in granulated form together with one or more coated tablets containing the O-acetylsalicylic acid or the physiologically acceptable salts thereof and, if required, both formulations are packed in a capsule.

4. Pharmaceutical combination as claimed in claims 1 and 2, characterised in that dipyridamole and/or the physiologically acceptable salts thereof together with an acid excipient are present in the form of pellets in a ratio of at least 1 val of the acid excipient to 1 mol of dipyridamole, these pellets being enclosed in a lacquer which is insoluble in gastric juices but soluble in intestinal juices, which releases an acid solution of the active substance in the gastrointestinal tract in a uniformly delayed manner, together with one or more coated tablets containing O-acetylsalicylic acid or the physiologically acceptable salts thereof and, if required, both formulations are packed in a capsule.

5. Pharmaceutical composition as claimed in claims 1 to 4, characterised in that it contains between 10 and 675 mg of dipyridamole and/or mopidamol and 1 and 150 mg of O-acetylsalicylic acid or the physiologically acceptable salts of these active substances, but preferably between 75 and 200 mg of dipyridamole and/or mopidamol and 5 to 80 mg of O-acetylsalicylic acid or the physiologically acceptable salts of these active substances.

6. Process for preparing pharmaceutical combinations containing dipyridamole and/or mopidamol or the physiologically acceptable salts thereof and O-acetylsalicylic acid or the physiologically acceptable salts thereof, at least one of the two components being protected from the other by a separating layer, characterised in that the pyrimido-pyrimidine component and the O-acetylsalicylic acid component are mixed together in a weight ratio of 8:1 or more, and if desired both components and previously provided with conventional carriers and/or excipients, and one or both of these components are added in the form of granulates, pellets or coated tablets and at least one of these components is provided with a coating which ensures simultaneous release of the active substances in the gastrointestinal tract.

7. Use of a pharmaceutical combination as claimed in claim 1 for preparing a pharmaceutical composition with an inhibitory effect on clot formation.

**Claims for the Contracting States: AT, ES, GR**

1. Process for preparing pharmaceutical combinations containing dipyridamole and/or mopidamol or the physiologically acceptable salts thereof and O-acetylsalicylic acid or the physiologically acceptable salts thereof, at least one of the two components being protected from the other by a separating layer, characterised in that the pyrimido-pyrimidine component and the O-acetylsalicylic acid component are mixed together in a weight ratio of 8:1 or more, and if desired both components are previously provided with conventional carriers and/or excipients, and one or both of these components are added in the form of granulates, pellets or coated tablets and at least one of these components is provided with a coating which ensures simultaneous release of the active substances in the gastrointestinal tract.

2. Process according to claim 1 for preparing an instant form, characterised in that dipyridamole or mopidamol pellets or granulates are coated with a lacquer which is insoluble in gastric juices but soluble in intestinal juices and these pellets or granulates are mixed with the O-acetylsalicylic acid.

3. Process according to claim 1 for preparing instant forms, characterised in that cores or tablets of O-acetylsalicylic acid are provided with an acetic acid-resistant coating which dissolves rapidly in gastric juices and are then mixed with dipyridamole or mopidamol granulates or pellets.

4. Process according to claims 1 and 3, characterised in that a dipyridamole or mopidamol granulate or pellets are packed into capsules together with one or more sugar-coated or film-coated tablets provided with a protective coating and containing the O-acetylsalicylic acid.

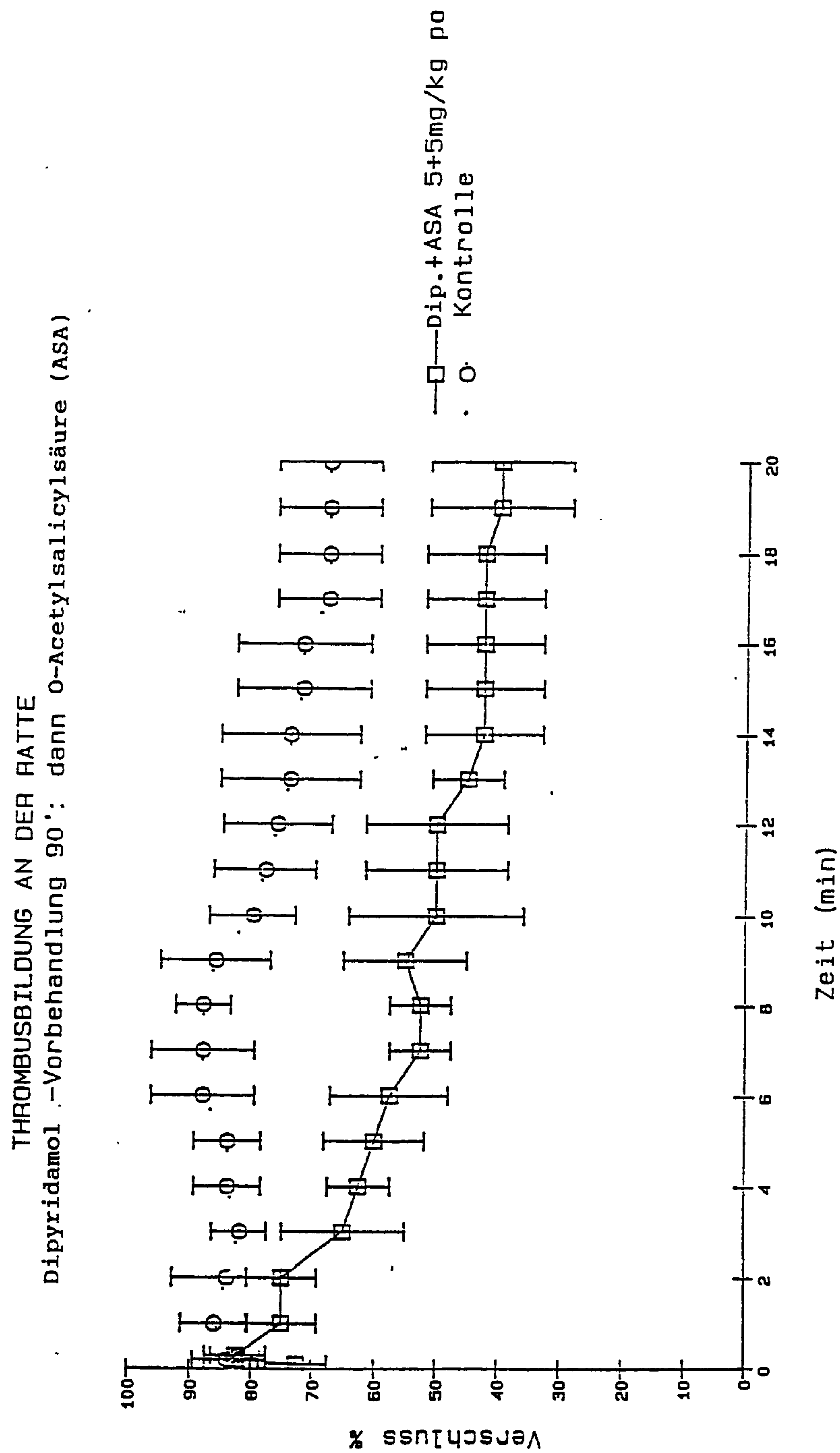
THROMBUSBILDUNG AN DER RATTE
Dipyridamol -Vorbehandlung 90': dann O-Acetylsalicylsäure (ASA)
Dip.+ASA 5+5mg/kg po
Kontrolle
Verschluss %
100 90 80 70 60 50 40 30 20 10 0
0 2 4 6 8 10 12 14 16 18 20
Zeit (min)

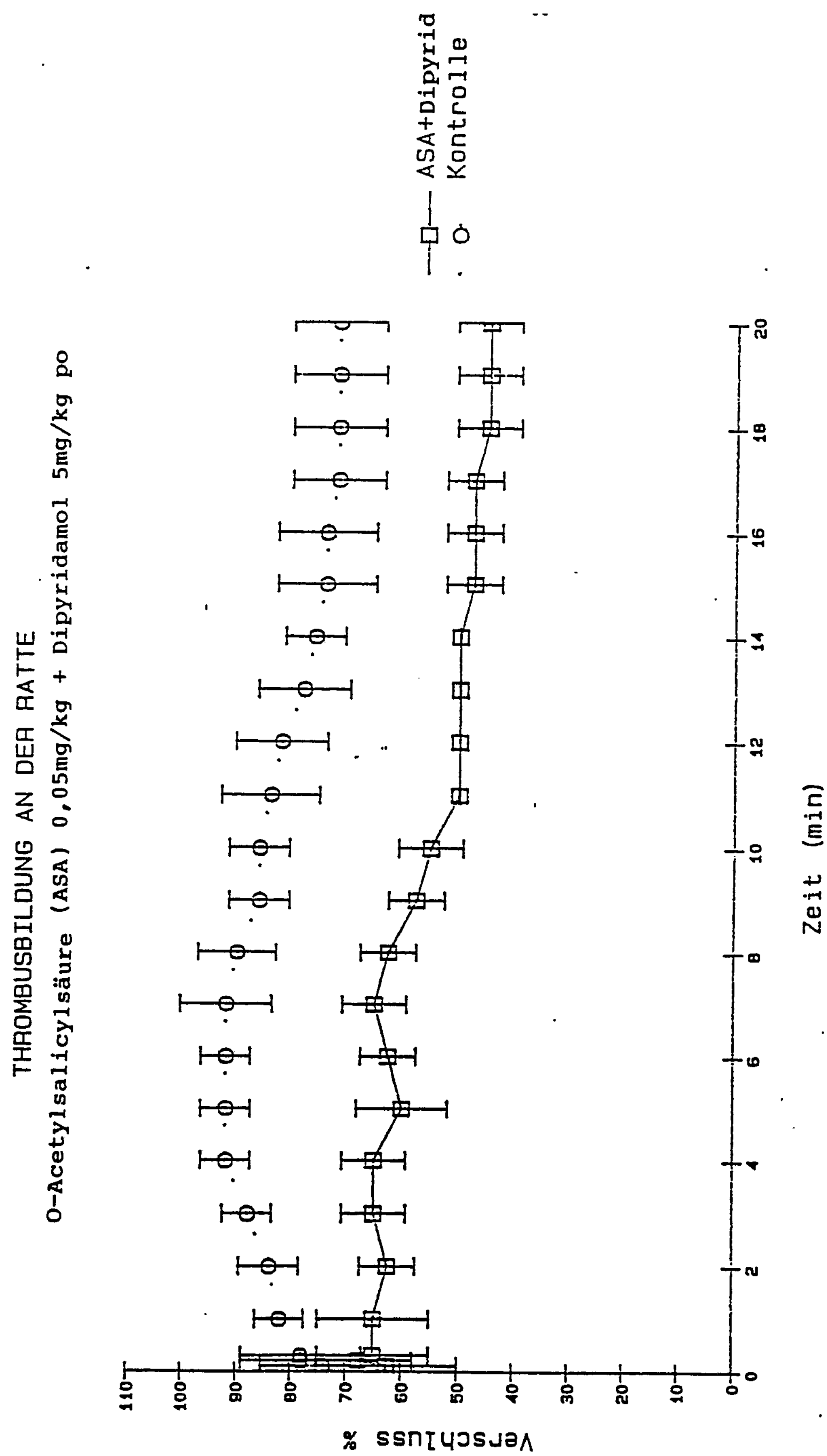
THROMBUSBILDUNG AN DER RATTE
O-Acetylsalicylsäure (ASA) 0,05mg/kg + Dipyridamol 5mg/kg po
ASA+Dipyrid
Kontrolle
Verschluss %
Zeit (min)
0
10
20
30
40
50
60
70
80
90
100
110
2
4
6
8
10
12
14
16
18
20

THROMBUSBILDUNG AN DER RATTE
Zeit: 10"-1'

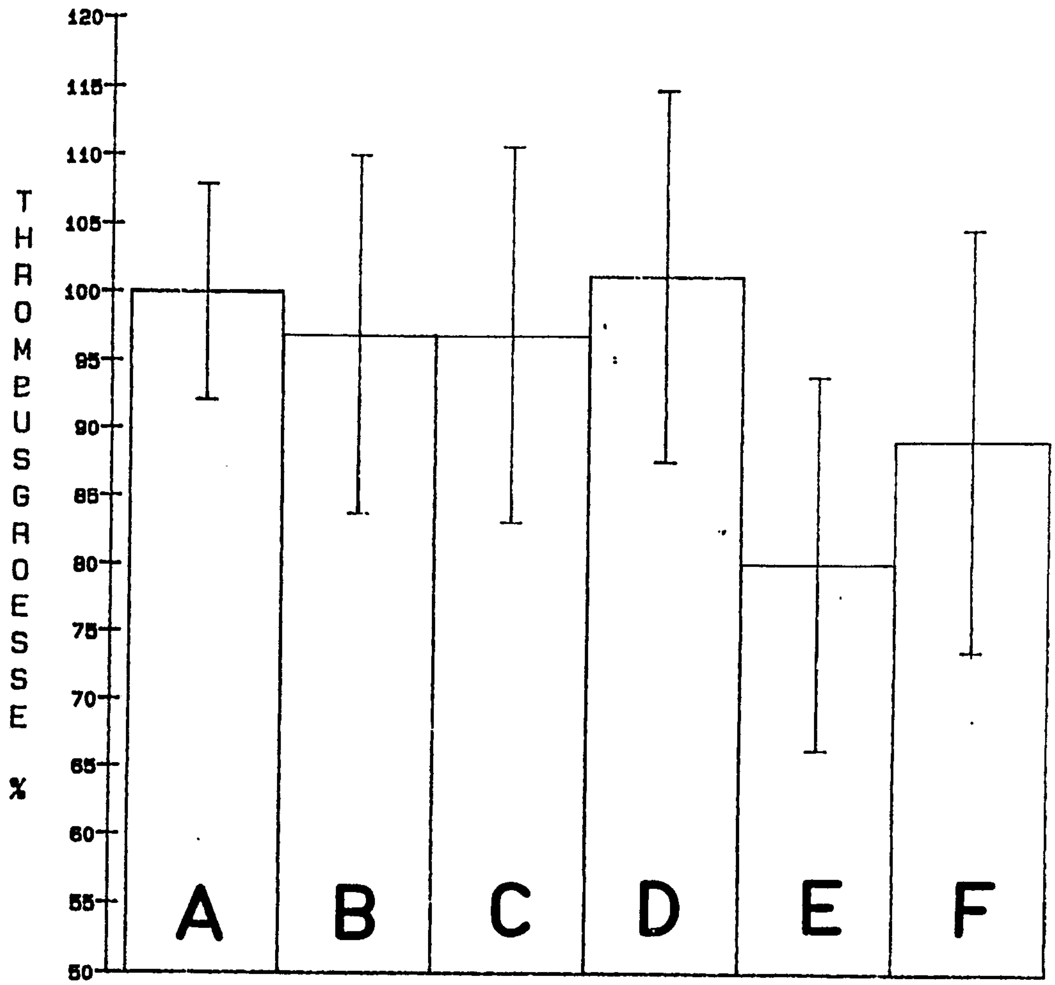

THROMBUSBILDUNG AN DER RATTE
Zeit: 2'-4'

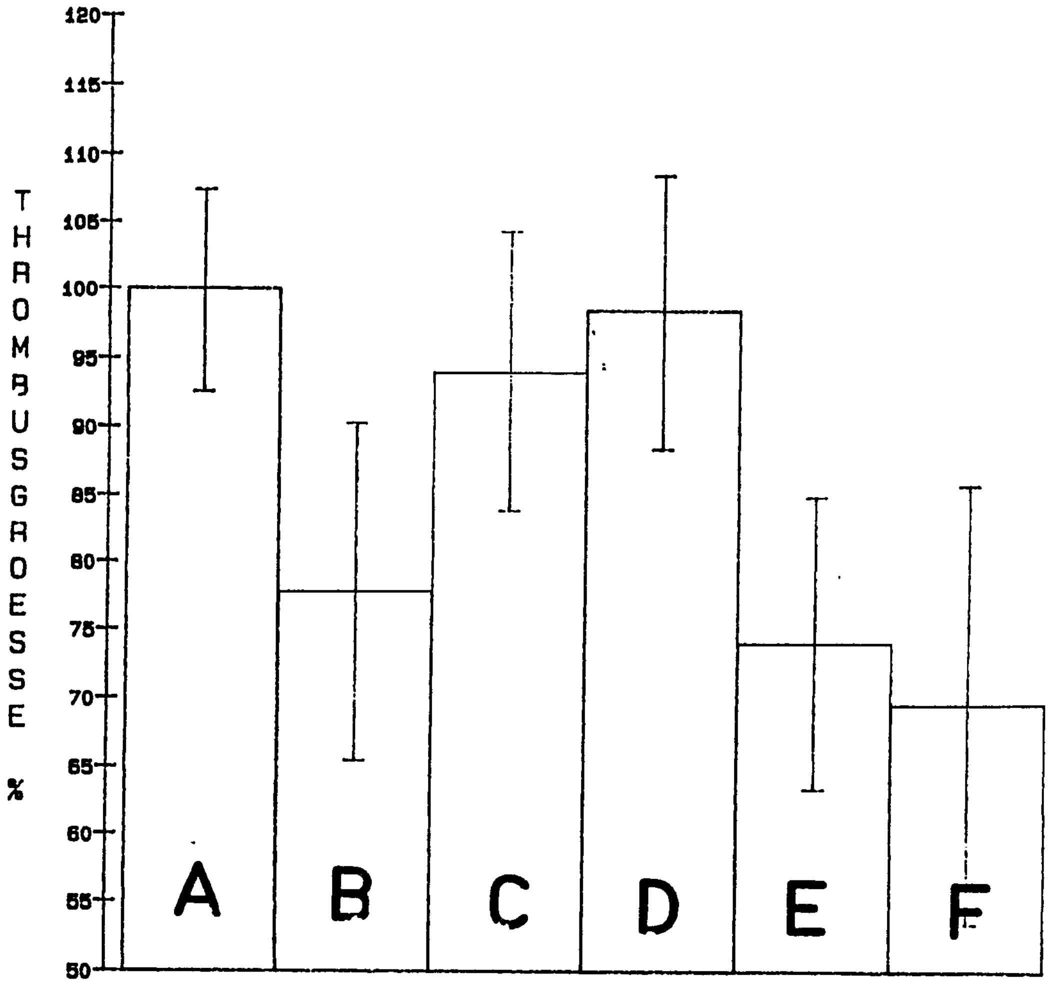

THROMBUSBILDUNG AN DER RATTE
Zeit: 5'-10'

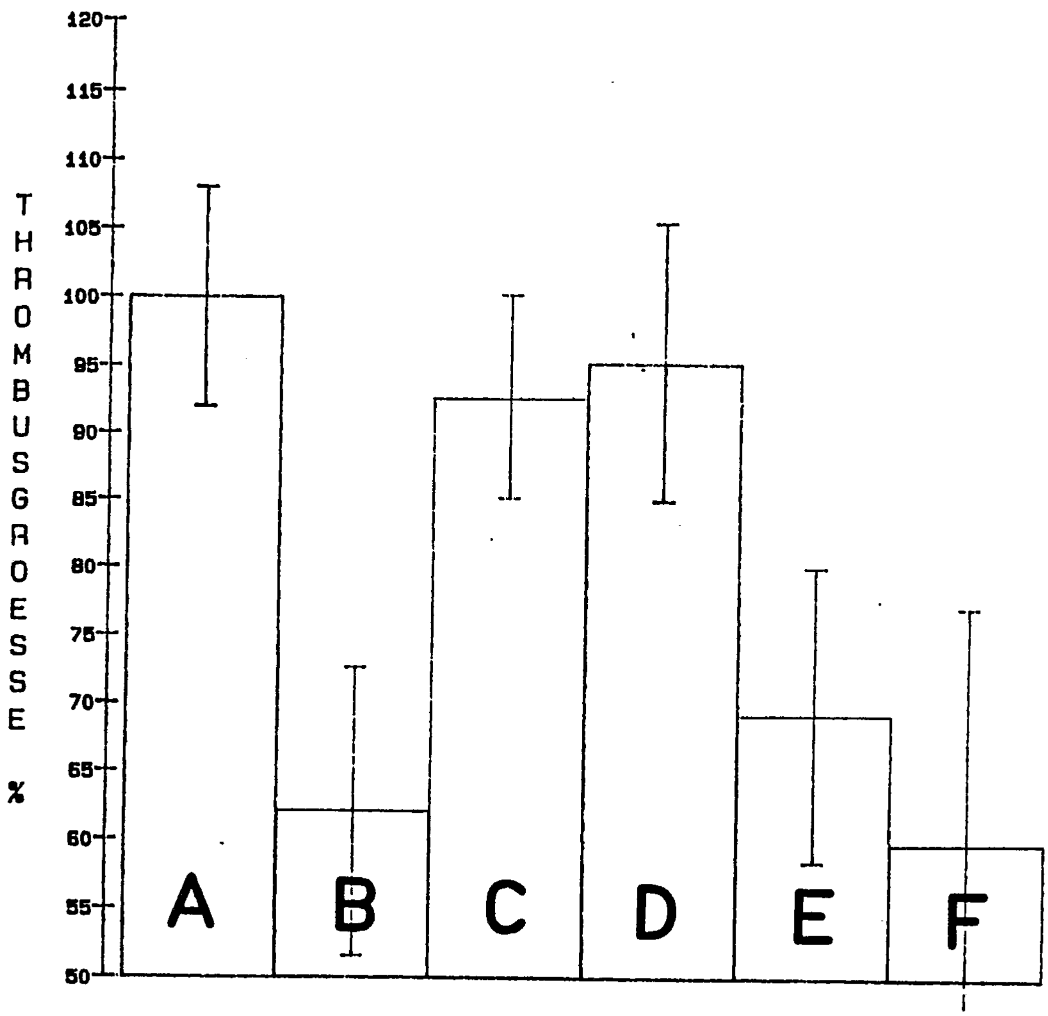

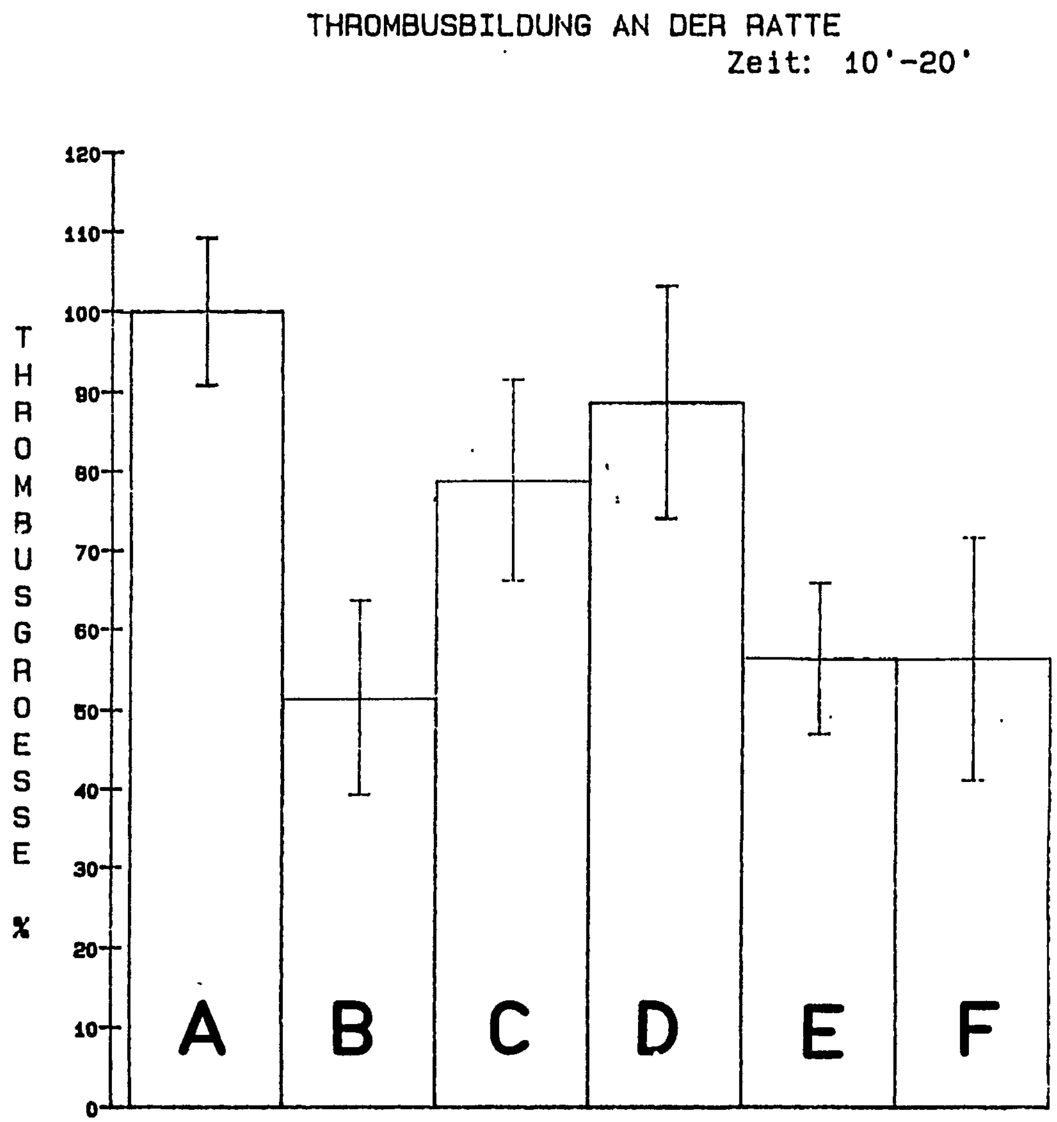
THROMBUSBILDUNG AN DER RATTE
Zeit: 10'-20'
THROMBUSGROESSE %
120
110
100
90
80
70
60
50
40
30
20
10
0
A
B
C
D
E
F

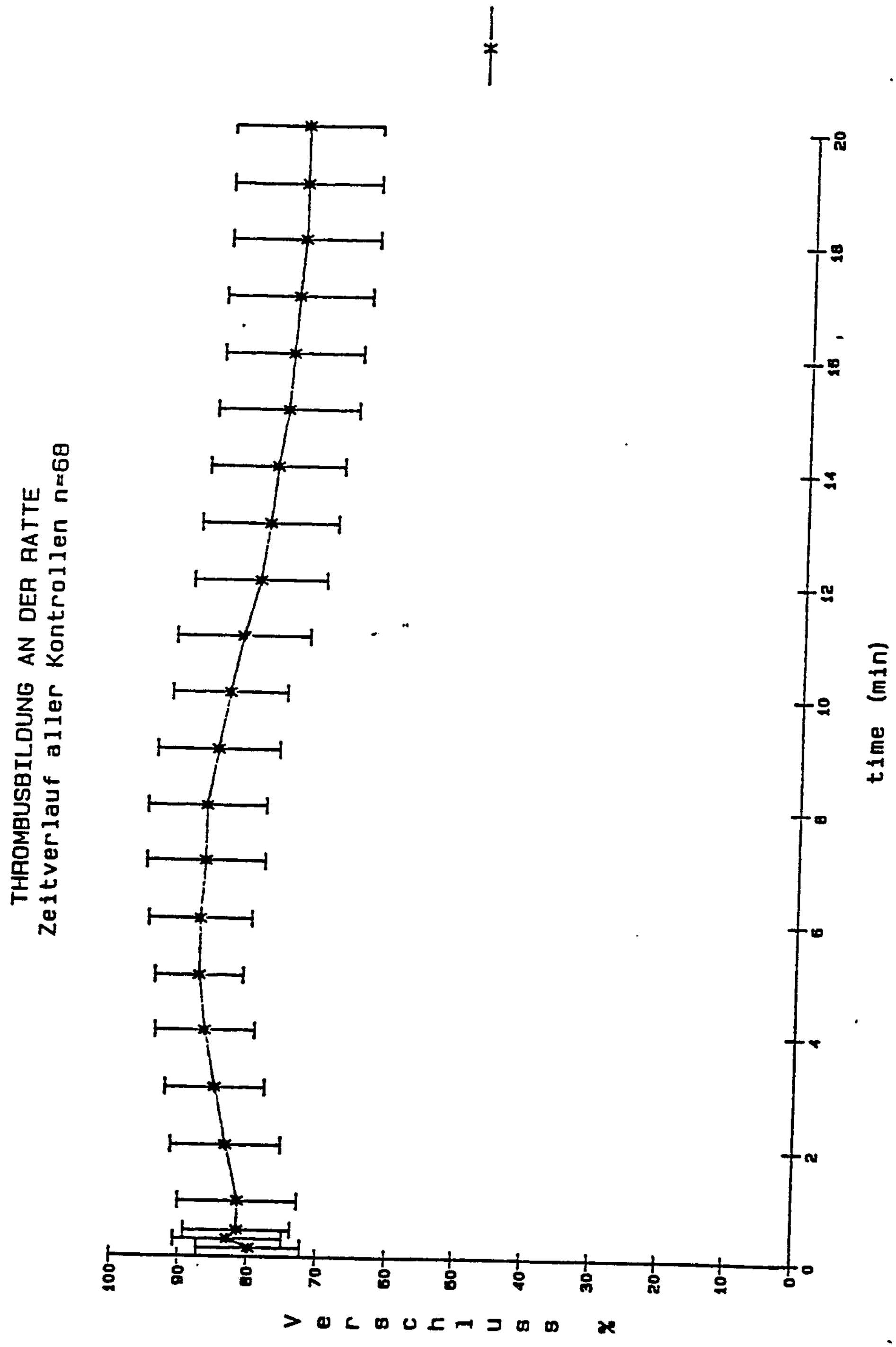
THROMBUSBILDUNG AN DER RATTE
Zeitverlauf aller Kontrollen n=68
Verschluss %
100
90
80
70
60
50
40
30
20
10
0
time (min)
0
2
4
6
8
10
12
14
16
18
20

O-Acetylsalicylsäure (ASA) 500 µg/kg + Dipyridamol 5 mg/kg i.mes.

n = 4

Rel. "free flow"

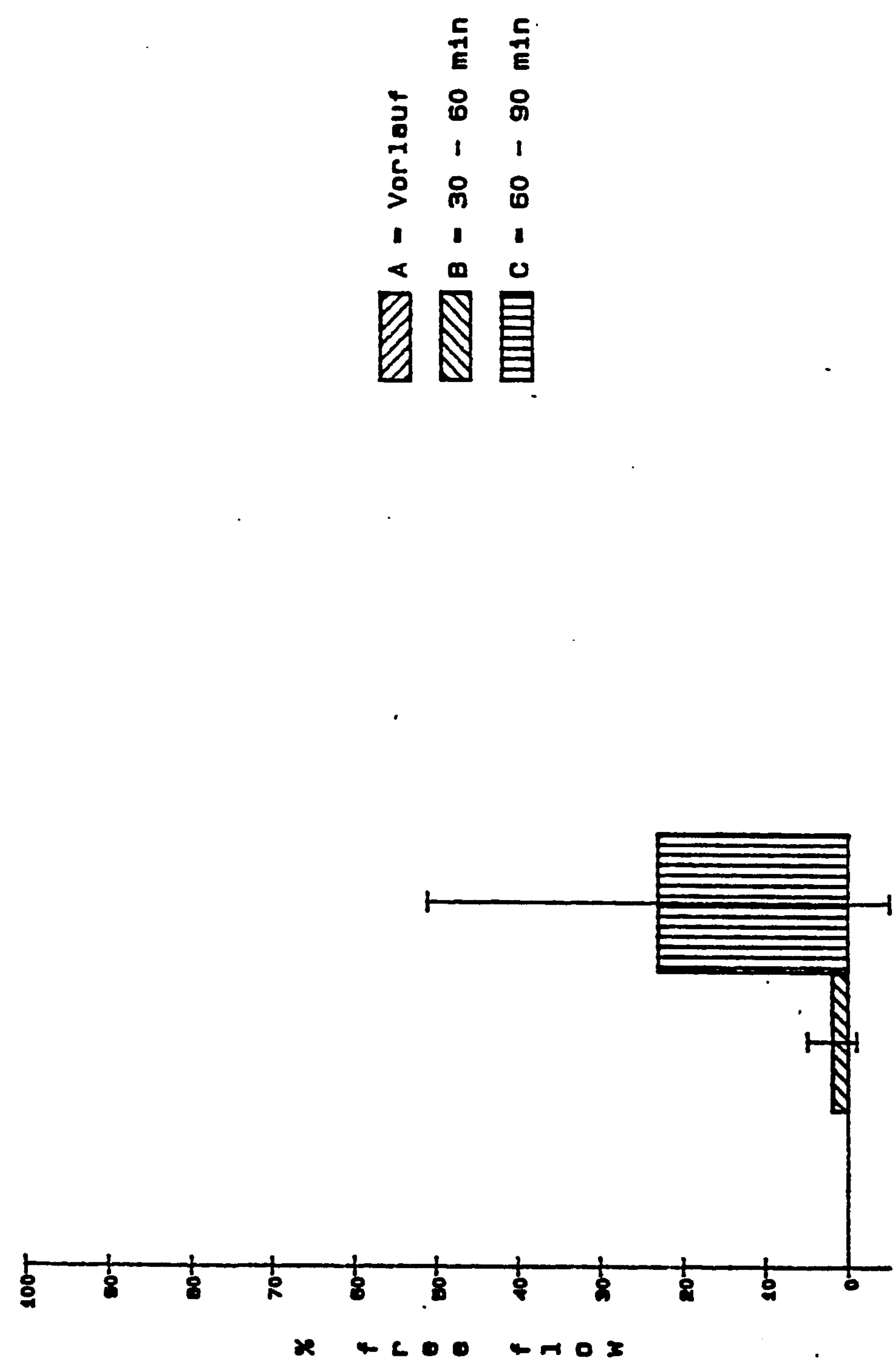

O-Acetylsalicylsäure (ASA) 50 µg/kg + Dipyridamol 5 mg/kg i.mes.

n = 4

Rel. "free flow"

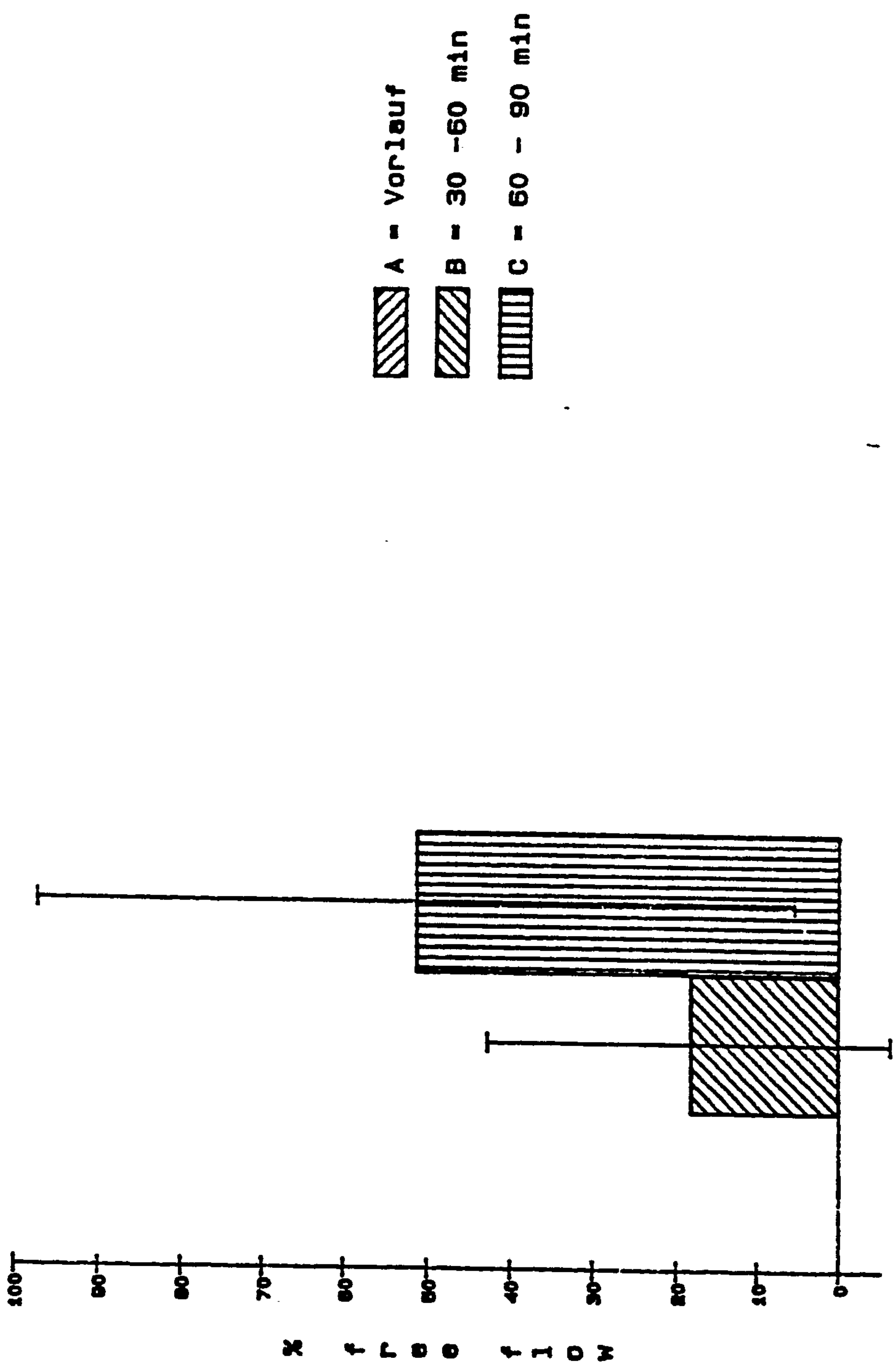

O-Acetylsalicylsäure (ASA) 100 µg/kg + Dipyridamol 5 mg/kg i.mes.

n = 4

Rel. "free flow"

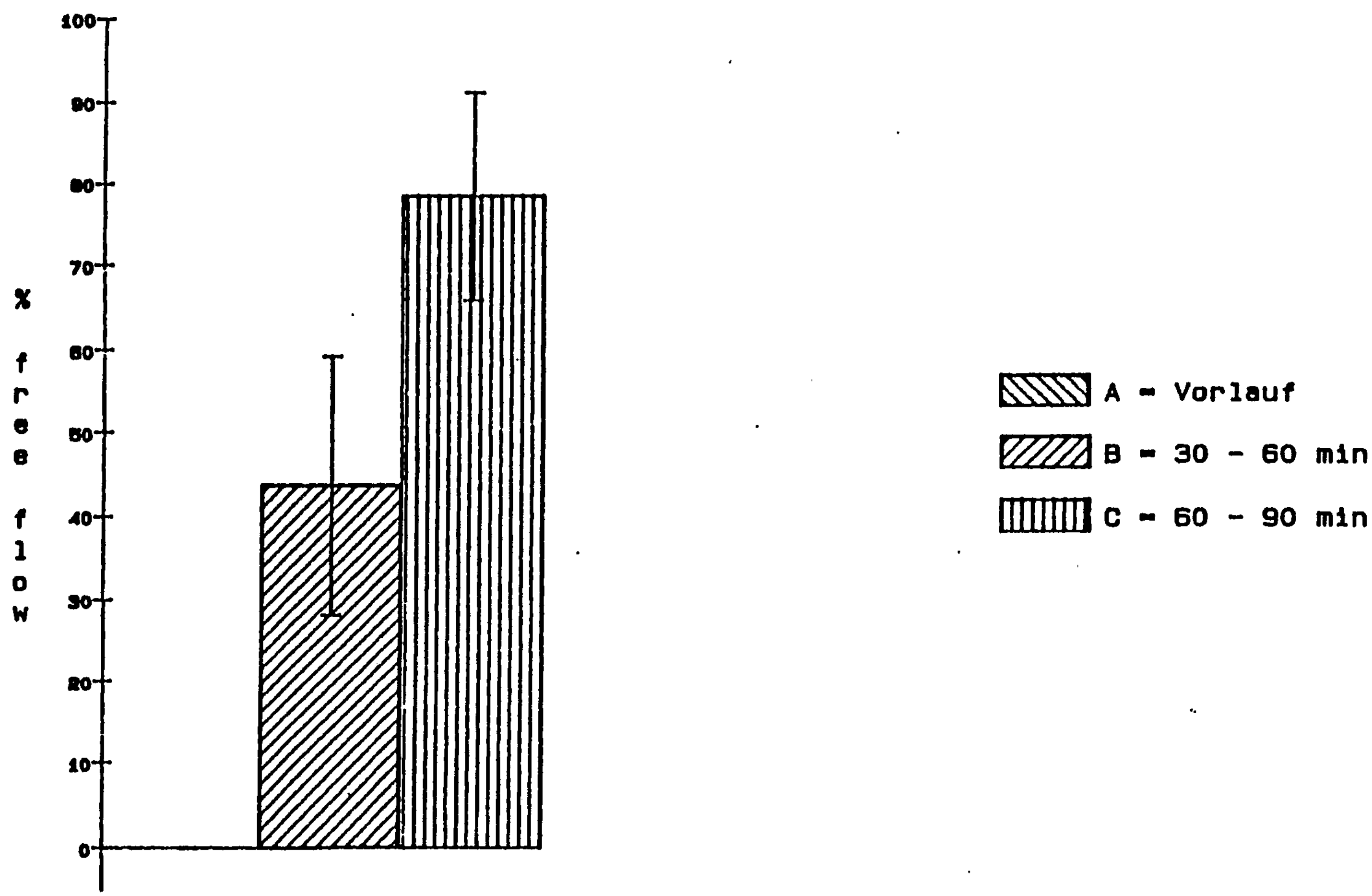

O-Acetylsalicylsäure (ASA) 5 mg/kg + Dipyridamol 1 mg/kg i.mes.

n = 4

Rel. "free flow"